# EUROPEAN PATENT APPLICATION

(11) **EP 4 663 201 A1**
(43) Date of publication of application: **17.12.2025**
(21) Application number: 24753166.8
(22) Date of filing: 29.01.2024
(51) Int. Cl.: A61K 39/35, A61K 9/06, A61K 36/185, A61K 39/36, A61K 47/61, A61P 37/08

(54) **FORMULATION FOR ALLERGEN IMMUNOTHERAPY**

(30) Priority: 08.02.2023 JP 2023017542
(71) Applicant: INSTITUTE OF SCIENCE TOKYO, Tokyo 152-8550 (JP); Wako Filter Technology Co., Ltd., Tokyo 101-0044 (JP)
(72) Inventor: KOMATSUZAKI, Keiko, Tokyo 152-8550 (JP); MIYAZAKI, Yasunari, Tokyo 152-8550 (JP); KAGESHIMA, Hiroki, Bando-shi, Ibaraki 306-0616 (JP); SEKINO, Yuki, Bando-shi, Ibaraki 306-0616 (JP)
(74) Representative: D Young & Co LLP
(86) International application number: PCT/JP2024/002583
(87) International publication number: WO 2024/166722

(57) **Abstract**

An object of the present invention is to provide an attenuated allergen that enables long-term retention of the allergen in mucosa to be performed relatively easily, conveniently, and safely while minimizing adverse events such as pain or induction of allergic symptoms, and is useful in allergen immunotherapy for allergic diseases such as pollinosis or pollen-food allergy syndrome. An allergen-polysaccharide conjugate is intranasally administered to an allergic disease patient such as a pollinosis patient or a pollen-food allergy syndrome patient.

## Description

### Technical Field

The present invention relates to a formulation for allergen immunotherapy and for intranasal administration to an allergic disease patient, comprising an allergen-polysaccharide conjugate as an active ingredient (hereinafter, also referred to as the "present formulation for allergen immunotherapy").

### Background Art

Pollen-food allergy syndrome (in the present specification, also referred to as "PFAS") is immediate-type food allergy that occurs when a patient sensitized to pollen eats a fruit or a vegetable having high homology of an amino acid sequence to a pollen-derived protein. The number of PFAS patients has increased drastically in school-aged children or older people in recent years. Most of the patients remain in oral allergy syndrome (in the present specification, also referred to as "OAS"), though some cases develop anaphylaxis. For PFAS patients, spontaneous remission is not usually expected, and a food responsible for allergy needs to be avoided over a lifetime.

The only one radical therapy for allergic diseases such as PFAS is allergen immunotherapy (in the present specification, also referred to as "AIT"). AIT is a treatment method aimed at preventing the progression of allergic response by administering an allergen responsible for a disease to an allergic disease patient and thereby weakening response to the allergen. AIT has heretofore focused on allergen immunotherapy by subcutaneous administration (subcutaneous immunotherapy). However, in addition to the subcutaneous immunotherapy, oral immunotherapy (in the present specification, also referred to as "OIT") and sublingual immunotherapy (in the present specification, also referred to as "SLIT") have also become known in recent years. For example, the development of a formulation suitable for SLIT is also underway (patent document 1).

In AIT, it is considered desirable to use a hypoallergenic allergen (i.e., an attenuated allergen) from the viewpoint of circumventing the risk of developing allergic symptoms including anaphylaxis. For example, a cedar pollen-derived protein conjugated with galactomannan, one of polysaccharides, by use of Maillard reaction has been reported to exhibit markedly reduced reactivity with human IgE (attenuation of the allergen) as compared with a cedar pollen-derived protein unconjugated with galactomannan (patent document 2). Also, use of a cedar pollen-derived protein-galactomannan conjugate to carry out OIT in cedar pollinosis patients has been reported to exert a symptom-improving effect in a short time (approximately 2 months) without causing adverse events (non-patent document 1). However, the intranasal administration of an allergen-polysaccharide conjugate and its effect in this case have not yet been known about AIT. Particularly, an effect of AIT (specifically, SLIT) on PFAS is limited (non-patent document 2), and AIT does not serve as a standard treatment method for PFAS in a current situation.

### Prior Art Documents

### Patent Documents

Patent Document 1: Japanese unexamined Patent Application Publication No. 2021-54738
Patent Document 2: Japanese unexamined Patent Application Publication No. 2006-340658

### Non-patent Documents

Non-patent Document 1: Murakami D, et al. Sci Rep. 2017. PMID: 28397833
Non-patent Document 2: Kinaciyan T, et al. J Allergy Clin Immunol. 2007 Apr; 119 (4): 937-43

### Summary of the Invention

### Object to be Solved by the Invention

An object of the present invention is to provide an attenuated allergen that enables long-term retention of the allergen in mucosa to be performed relatively easily, conveniently, and safely while minimizing adverse events such as pain or induction of allergic symptoms, and is useful in allergen immunotherapy for allergic diseases such as pollinosis or pollen-food allergy syndrome.

### Means to Solve the Object

In the course of pursuing diligent studies to solve the object, the present inventors have confirmed using an *in vitro* system that a conjugate of an allergen *Betulaceae* pollen and a polysaccharide galactomannan (*Betulaceae* pollen-galactomannan conjugate) exhibits reduced binding activity against Betulaceae-specific IgE (i.e., is hypoallergenic) while maintaining binding activity against *Betulaceae-specific* IgG or IgA. As a result of conducting an intranasal administration test on model animals and humans using the *Betulaceae* pollen-galactomannan conjugate, the present inventors have then found that dendritic cells can present the *Betulaceae* pollen in the *Betulaceae* pollen-galactomannan conjugate as an antigen, as in a usual *Betulaceae* pollen antigen, while allergic symptoms are minimized; and *Betulaceae-specific* IgG and IgA in saliva or blood are increased. The present inventors have also confirmed that long-term retention of *Betulaceae* pollen in human nasal mucosa can be performed relatively easily, conveniently, and safely without involving pain or induction of allergic symptoms. The present inventors have further confirmed that the *Betulaceae* pollen-galactomannan conjugate, when intranasally administered to a patient with apple allergy caused by *Betulaceae* pollen sensitization (PFAS patient), can increase Betulaceae-specific IgG in blood while minimizing allergic symptoms, and improves allergic symptoms to *Betulaceae* pollen and apple, resulting in drastic increase in the amount of an orally ingestible apple. The present invention has been completed on the basis of these findings.

Specifically, the present invention is as follows.
[1] A formulation for allergen immunotherapy, comprising an allergen-polysaccharide conjugate, wherein the formulation for allergen immunotherapy is intranasally administered to an allergic disease patient.
[2] The formulation for allergen immunotherapy according to [1], wherein the formulation for allergen immunotherapy is an ointment formulation.
[3] The formulation for allergen immunotherapy according to [1] or [2], wherein the polysaccharide is galactomannan.
[4] The formulation for allergen immunotherapy according to any one of [1] to [3], wherein the allergen is a pollen-derived allergen.
[5] The formulation for allergen immunotherapy according to [4], wherein the allergic disease patient is a pollinosis patient or a pollen-food allergy syndrome patient.
[6] The formulation for allergen immunotherapy according to [4] or [5], wherein the pollen is *Fagales* pollen.
[7] The formulation for allergen immunotherapy according to [6], wherein the *Fagales* pollen is *Betulaceae* pollen or *Fagaceae* pollen.
[8] The formulation for allergen immunotherapy according to [7], wherein the *Betulaceae* pollen is white birch pollen or alder pollen.

Examples of other embodiments of the present invention can include:
allergen immunotherapy comprising the step of intranasally administering a formulation for allergen immunotherapy to an allergic disease patient, the formulation for allergen immunotherapy comprising an allergen-polysaccharide conjugate;
an allergen-polysaccharide conjugate for use in allergen immunotherapy, the allergen-polysaccharide conjugate being intranasally administered to an allergic disease patient; and
use of an allergen-polysaccharide conjugate for producing a formulation for allergen immunotherapy, the formulation for allergen immunotherapy being intranasally administered to an allergic disease patient.

### Effect of the Invention

According to the present invention, long-term retention of attenuated allergen in mucosa can be performed relatively easily, conveniently, and safely while adverse events such as pain or induction of allergic symptoms are minimized. Therefore, allergen immunotherapy can be carried out for a long period in a stress-free and painless manner even in infants and young children, expectant and nursing mothers, or breast-feeding patients. Thus, allergic symptoms in allergic disease patients such as pollinosis patients or pollen-food allergy syndrome patients can be effectively improved.

AIT using the present formulation for allergen immunotherapy is unlikely to induce allergic symptoms, as compared with conventional AIT. Therefore, the present formulation for allergen immunotherapy can be intermittently administered as maintenance therapy for several years to several tens of years. Daily administration of an allergen in AIT generally has the risk of reducing adherence. Nonetheless, in AIT using the present formulation for allergen immunotherapy, even if adherence is reduced, the allergen-polysaccharide conjugate contained in the present formulation for allergen immunotherapy, because of being an attenuated allergen, is unlikely to cause allergic symptoms upon resuming AIT using the present formulation for allergen immunotherapy, and is therefore considered able to secure safety even for AIT at locations other than medical settings (e.g., patient's home). Furthermore, unlike conventional AIT, AIT using the present formulation for allergen immunotherapy eliminates the need of finely setting an optimum dose for every patient and gradually increasing the dose over months until a maintenance dose is reached. Therefore, a therapeutically effective amount of the allergen-polysaccharide conjugate can be rapidly administered without mistaking the dose. Thus, the complication of conventional AIT can be reduced.

### Brief Description of Drawings

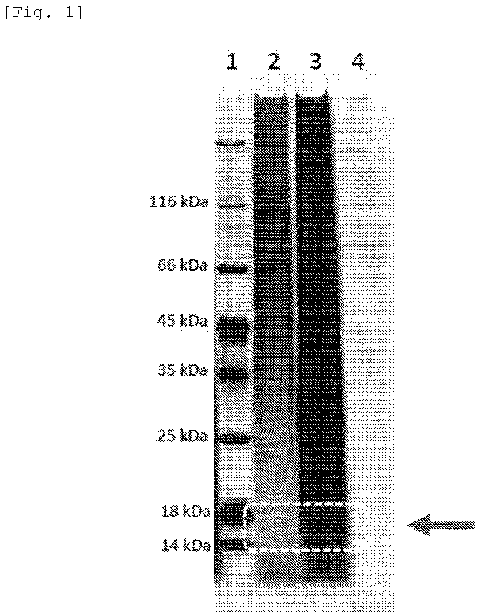

[Figure 1] Figure 1 is a diagram showing results of performing polyacrylamide electrophoresis for 3 types of samples ("white birch pollen-galactomannan conjugate" [lane 2 in the drawing], "white birch pollen crude extract antigen-galactomannan mixed powder" [lane 3 in the drawing], and galactomannan [lane 4 in the drawing]) and a molecular weight marker (lane 1 in the drawing). The arrow in the drawing depicts a band derived from Bet v1, a major antigen of white birch pollen.

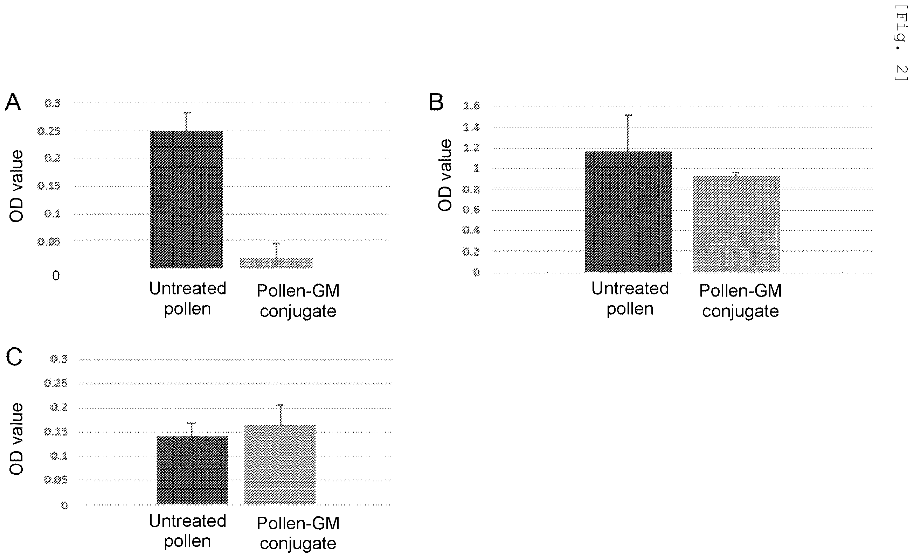

[Figure 2] Figure 2 is a diagram showing results of performing an ELISA (enzyme-linked immunosorbent assay) method by adding PFAS patient-derived serum to wells on which four types of antigens ("alder pollen crude extract antigen-galactomannan mixed powder" ["untreated pollen" in Figures 2A and 2B] and "alder pollen-galactomannan conjugate" ["pollen-GM conjugate" in Figures 2A and 2B], and "white birch pollen crude extract antigen-galactomannan mixed powder" ["untreated pollen" in Figure 2C] and "white birch pollen-galactomannan conjugate" ["pollen-GM conjugate" in Figure 2C]) are immobilized and then adding thereto 3 types of antibodies (HRP [horseradish peroxidase]-labeled anti-human IgE antibody [Figure 2A], HRP-labeled anti-human IgG antibody [Figure 2B], and HRP-labeled anti-human IgA antibody [Figure 2C]).

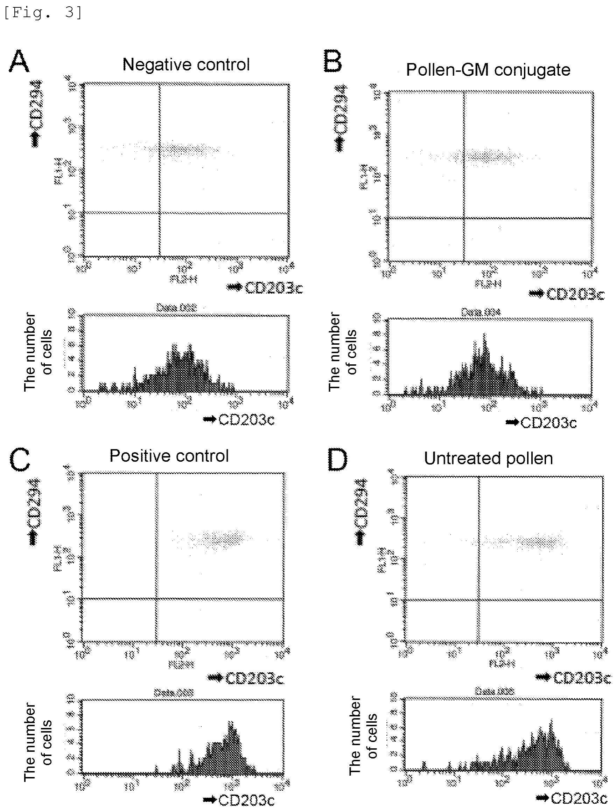

[Figure 3] Figure 3 is a diagram showing results of performing a basophil activation test (BAT) using Allergenicity kit (manufactured by Beckman Coulter, Inc.) for PFAS patient-derived blood incubated in the presence of 2 types of antigens ("white birch pollen crude extract antigen-galactomannan mixed powder" ["untreated pollen" in Figure 3D] and "white birch pollen-galactomannan conjugate" ["pollen-GM conjugate" in Figure 3B]) and PFAS patient-derived blood incubated in the presence of 2 types of controls (positive control anti-IgE antibody [Figure 3C] and negative control phosphate-buffered saline [PBS] [Figure 3A]).

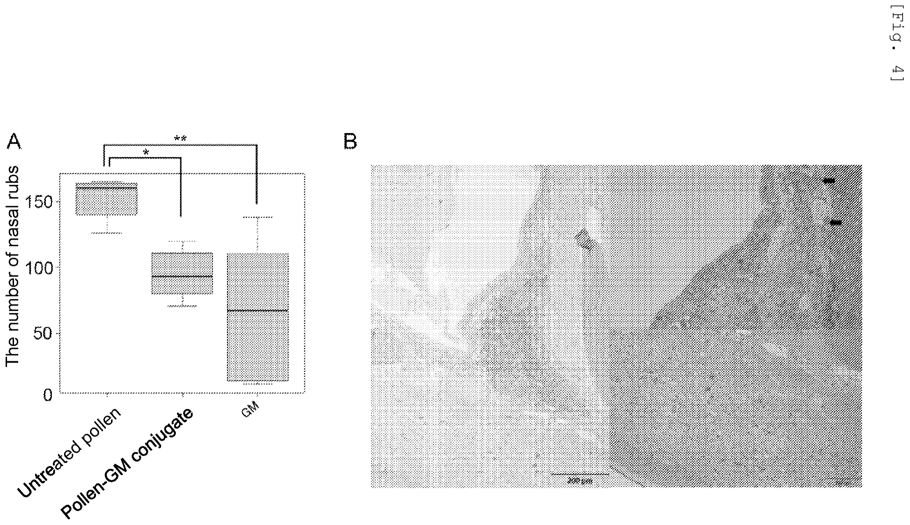

[Figure 4] Figure 4A is a diagram showing results of intranasally administering 3 types of antigens ("white birch pollen crude extract antigen-galactomannan mixed powder" [untreated pollen], "white birch pollen-galactomannan conjugate" [pollen-GM conjugate], and galactomannan [GM]) to mice sensitized to a white birch pollen crude extract antigen and then measuring the number of nasal rubs. In the drawing, "*" and "**" each denote having statistically significant difference by the Tukey's test (p < 0.05 and p < 0.01). Figure 4B is a diagram showing results of performing an immunohistological staining method using an anti-Bet v1 antibody and an anti-CD11c antibody for a nasal tissue specimen of the mouse given the intranasally administered "white birch pollen-galactomannan conjugate".

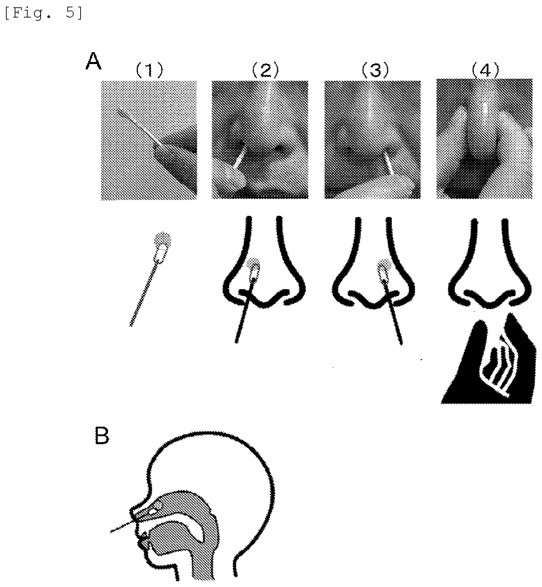

[Figure 5] Figure 5A is a sequence of photographs of intranasal administration to 2 types of groups ("white birch pollen-galactomannan conjugate"-containing ointment formulation administration group and placebo administration group) (upper) and a schematic view thereof (lower). Figure 5B is a schematic view of intranasal administration to the 2 types of groups.

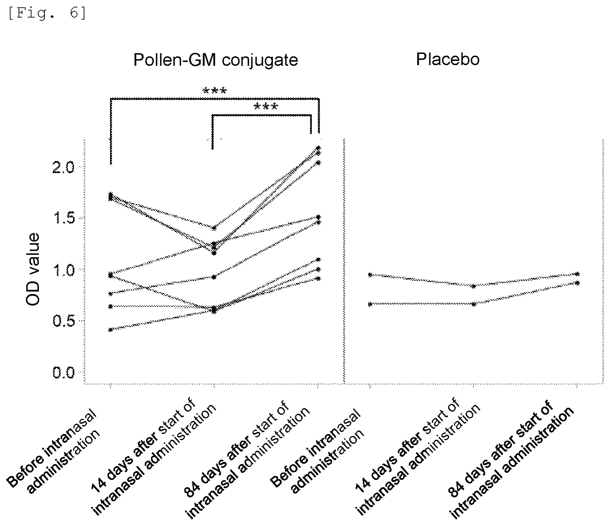

[Figure 6] Figure 6 is a diagram showing results of measuring a serum white birch-specific IgG concentration in 2 types of groups ("white birch pollen-galactomannan conjugate"-containing ointment formulation administration group ["pollen-GM conjugate" in the drawing] and placebo administration group ["placebo" in the drawing]) before intranasal administration, 14 days after the start of intranasal administration, and 84 days after the start of intranasal administration. In the drawing, "***" denotes having statistically significant difference by the paired T test (p < 0.001).

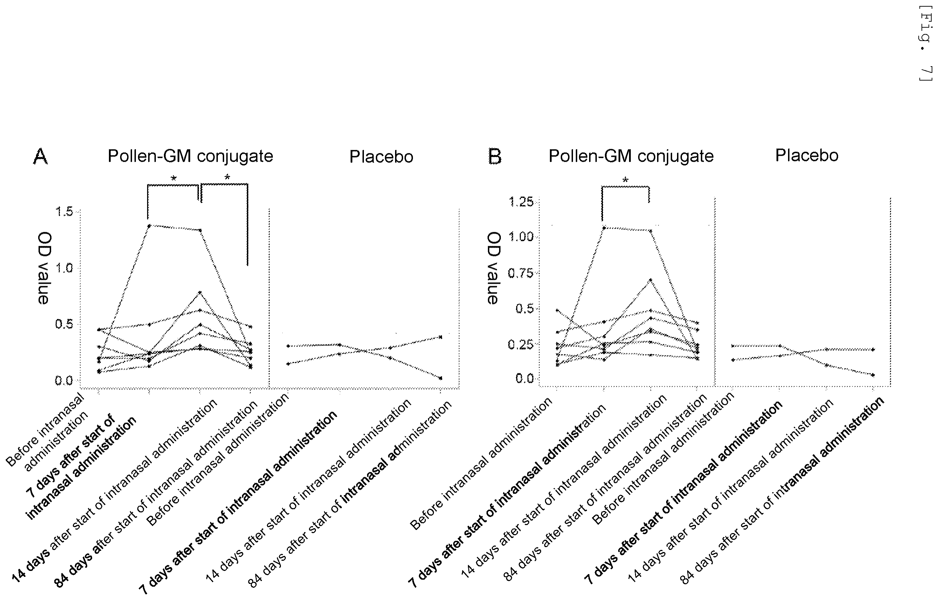

[Figure 7] Figure 7 is a diagram showing results of measuring a saliva white birch-specific IgA concentration (Figure 7A) and a saliva white birch-specific IgG concentration (Figure 7B) in 2 types of groups ("white birch pollen-galactomannan conjugate"-containing ointment formulation administration group ["pollen-GM conjugate" in the drawing] and placebo administration group ["placebo" in the drawing]) before intranasal administration, 7 days after the start of intranasal administration, 14 days after the start of intranasal administration, and 84 days after the start of intranasal administration. In the drawing, "*" denotes having statistically significant difference by the paired T test (p < 0.05).

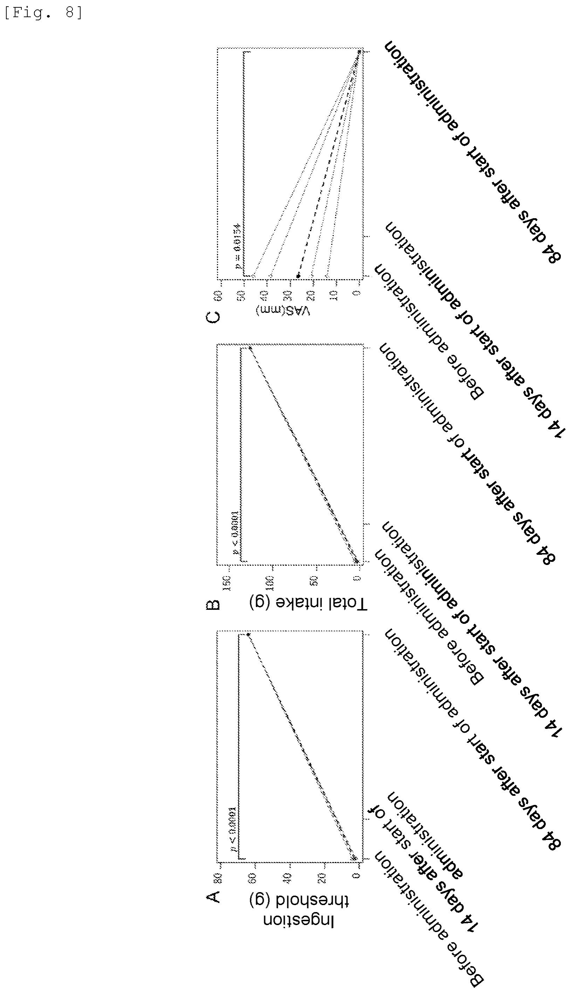

[Figure 8] Figure 8 is a diagram showing results of performing an oral challenge test with raw apple for 5 test subjects (patients with apple allergy caused by *Betulaceae* pollen sensitization) before intranasal administration of a "white birch pollen-galactomannan conjugate"-containing ointment formulation, 14 days after the start of intranasal administration, and 84 days after the start of intranasal administration and analyzing a weight threshold of apple that induced oral allergic symptoms (hereinafter, also referred to as an "ingestion threshold") (Figure 8A) and a total intake of apple that was able to be orally ingested (hereinafter, also referred to as a "total intake") (Figure 8B), and a visual analog scale (VAS) therein (Figure 8C). The solid line depicts each patient, and the dotted line depicts an average value of patients.

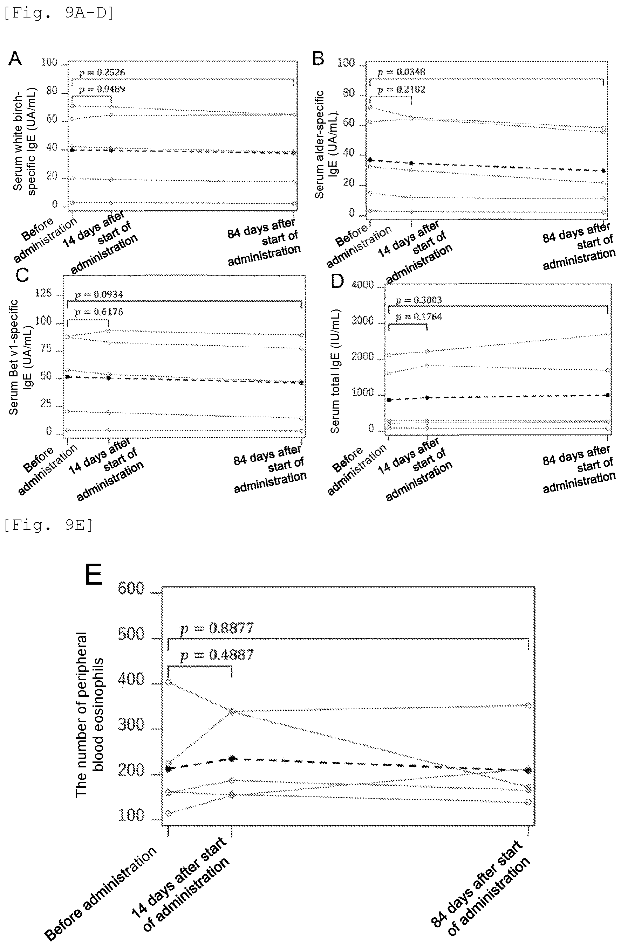

[Figures 9A-9D] Figures 9A to 9D are diagrams showing results of measuring 4 types of IgE concentrations in serum (serum white birch-specific IgE concentration [Figure 9A], serum alder-specific IgE concentration [Figure 9B], serum Bet v1-specific IgE concentration [Figure 9C], and serum total IgE concentration [Figure 9D]) for 5 test subjects (patients with apple allergy caused by *Betulaceae* pollen sensitization) before intranasal administration of a "white birch pollen-galactomannan conjugate"-containing ointment formulation, 14 days after the start of intranasal administration, and 84 days after the start of intranasal administration. The solid line depicts each patient, and the dotted line depicts an average value of patients.
[Figure 9E] Figure 9E is a diagram showing results of measuring the number of peripheral blood eosinophils for 5 test subjects (patients with apple allergy caused by *Betulaceae* pollen sensitization) before intranasal administration of a "white birch pollen-galactomannan conjugate"-containing ointment formulation, 14 days after the start of intranasal administration, and 84 days after the start of intranasal administration. The solid line depicts each patient, and the dotted line depicts an average value of patients.

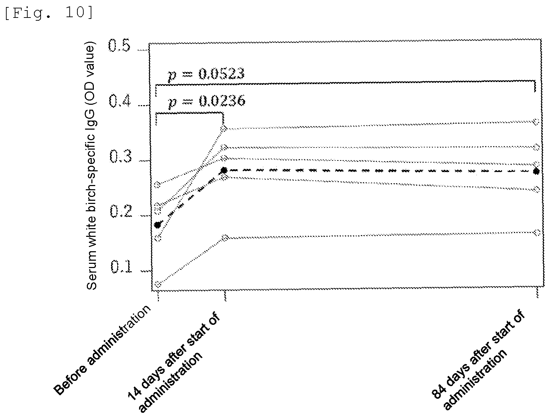

[Figure 10] Figure 10 is a diagram showing results of measuring a serum white birch-specific IgG concentration for 5 test subjects (patients with apple allergy caused by *Betulaceae* pollen sensitization) before intranasal administration of a "white birch pollen-galactomannan conjugate"-containing ointment formulation, 14 days after the start of intranasal administration, and 84 days after the start of intranasal administration. The solid line depicts each patient, and the dotted line depicts an average value of patients.

### Mode of Carrying Out the Invention

The formulation for allergen immunotherapy of the present invention is an agent containing an allergen-polysaccharide conjugate (i.e., a conjugate of an allergen and a polysaccharide) defined by the purpose of being "used in allergen immunotherapy" and the administration mode of "intranasal administration to an allergic disease patient" (i.e., the present formulation for allergen immunotherapy). The present formulation for allergen immunotherapy may be used alone as a medicament (formulation) and may be further mixed with an additive and used in the form of a composition (pharmaceutical composition).

The dosage form of the present formulation for allergen immunotherapy can be any form that permits intranasal administration. Examples thereof can include a nasal drop, a spray formulation, an aerosol formulation, an ointment formulation, a cream formulation, a lotion formulation, a liniment formulation, a cataplasm formulation, a plaster formulation, a patch formulation, an emplastrum formulation, a gel formulation, a liquid formulation, a tape formulation, a powder formulation, and a granule formulation. Preferred examples thereof can include an ointment formulation. The formulation can be prepared into a desired dosage form in accordance with a usual method described in the General Rules for Preparations of the Japanese Pharmacopoeia using an additive, a base, and the like suitable for each dosage form.

When the dosage form is an ointment formulation or a cream formulation, for example, an oleaginous base or an emulsion base can be used as a base.

Examples of the oleaginous base can include: a hydrocarbon (e.g., petrolatum [e.g., yellow petrolatum, white petrolatum, and Propeto; the same holds true for the description below], squalene, squalane, and Plastibase); a higher alcohol (e.g., lauryl alcohol, tridecyl alcohol, myristyl alcohol, and pentadecyl alcohol); a higher fatty acid (e.g., caproic acid, enanthic acid, caprylic acid, pelargonic acid, capric acid, undecylic acid, and lauric acid); a higher fatty acid ester (e.g., myristyl palmitate, stearyl stearate, myristyl myristate, and seryl lignocerate); a glycol (e.g., ethylene glycol, diethylene glycol, and propylene glycol); a plant oil (e.g., a camellia oil, castor oil, olive oil, cacao oil, and coconut oil); and an animal oil (e.g., mink oil, egg yolk oil, squalane, and squalene). One of these oleaginous bases may be used singly, or two or more thereof may be used in combination.

Examples of the emulsion base can include: an oil-in-water base (e.g., a base prepared by emulsifying and dispersing a component such as lanoline, propylene glycol, stearyl alcohol, petrolatum, silicone oil, liquid paraffine, glyceryl monostearate, or polyethylene glycol in the presence or absence of a surfactant); a water-in-oil base (e.g., a base prepared by emulsifying and dispersing a component such as petrolatum, higher aliphatic alcohol, or liquid paraffine by the addition of water in the presence of a nonionic surfactant); and a suspended base (e.g., an aqueous base prepared into a gel form by adding a suspending agent such as starch, glycerin, highly viscous carboxymethylcellulose, or a carboxyvinyl polymer to water). One of these emulsion bases may be used singly, or two or more thereof may be used in combination.

Examples of the additive can include a formulation ingredient such as a pH adjuster, a preservative, a flavoring agent, a dispersant, a wetting agent, a stabilizer, an antiseptic, a suspending agent, and a surfactant. Examples of the formulation ingredient can specifically include water, saline, an animal-derived fat and oil, a plant oil, lactose, starch, gelatin, crystalline cellulose, gum, talc, magnesium stearate, hydroxypropylcellulose, polyalkylene glycol, polyvinyl alcohol, and glycerin.

As used herein, the "allergen" means an antigen responsible for an allergic disease (allergic response) and usually includes a protein or a polypeptide constituting the protein. The allergen includes a naturally occurring allergen and a treated product thereof as well as an allergen artificially synthesized by use of a gene recombination technique or the like.

Examples of the allergen can include: an allergen derived from pollen such as *Fagales* pollen (e.g., *Betulaceae* pollen [e.g., alder pollen and white birch pollen], *Fagaceae* pollen [e.g., *Quercus serrata* pollen, Japanese chestnut pollen, and beech pollen]), *Cupressaceae* pollen (e.g., cedar pollen and Japanese cypress pollen), *Poaceae* pollen (e.g., timothy grass pollen and orchard grass pollen), and *Asteraceae* pollen (e.g., ragweed pollen and wormwood pollen); an allergen derived from an insect such as silkworm, mite, honeybee, hornet, ant, and cockroach; an allergen derived from a fungus such as *Alternaria, Aspergillus,* botulism, candida, *Cephalosporium, Curvularia, Epicoccum, Epidermophyton, Fusarium, Helminthosporium,* chain *Cladosporium,* mucor, *Penicillium, Phoma, Pullularia pullulans,* and rhizopus; an allergen derived from an animal such as a dog, a cat, and a bird; an allergen derived from a food such as chicken egg, soybean, bean sprout, peanut, cashew nut, buckwheat, wheat, seafood (e.g., cod, shrimp, and crab), meat (e.g., beef, pork, and chicken meat), apple, peach, pear, and cherry; and natural rubber. A pollen-derived allergen is preferred, a *Fagales* pollen-derived allergen is more preferred, a *Betulaceae* pollen-derived allergen or a *Fagaceae* pollen-derived allergen is further preferred, and a white birch pollen-derived allergen or an alder pollen-derived allergen is still further preferred.

As used herein, the "polysaccharide" means a sugar composed of monosaccharides polymerized through glycoside bonds. The molecular weight of the polysaccharide is usually in the range of 10000 to 1000000 (e.g., 10000 to 800000, 10000 to 600000, 10000 to 400000, 30000 to 800000, 30000 to 600000, 30000 to 400000, 100000 to 800000, 100000 to 600000, or 100000 to 400000).

Examples of the polysaccharide can include xyloglucan, chitosan, alginic acid, galactomannan, dextran, pullulan, and cellulose and can preferably include galactomannan.

The weight ratio between the allergen and the polysaccharide in the "allergen-polysaccharide conjugate" is not particularly limited and is usually in the range of 100:1 to 1:100 (e.g., 60:1 to 1:60, 30:1 to 1:30, 1:1 to 1:30, 1:1 to 1:20, 1:1 to 1:16, 1:1 to 1:13, 1:2 to 1:30, 1:2 to 1:20, 1:2 to 1:16, 1:2 to 1:13, 1:3 to 1:30, 1:3 to 1:20, 1:3 to 1:16, or 1:3 to 1:13).

The "allergen-polysaccharide conjugate" can be prepared by use of a conventional method for conjugating a protein with a sugar. For example, a mixture of a powder of the allergen and a powder of the polysaccharide can be incubated for 2 days to 42 days under conditions involving 50 to 70°C and a humidity of 60 to 80% so that Maillard reaction occurs to prepare a conjugate.

As used herein, the "allergic disease patient" means a subject (patient) in which an antibody is formed *in vivo* by ingestion of or contact with an allergen and antigen-antibody reaction occurs by reingestion of or recontact with the same allergen or a structurally similar allergen thereas, causing at least one allergic symptom (e.g., a respiratory symptom such as nasal discharge, sneeze, nasal congestion, and cough; a digestive symptom such as stomach ache, diarrhea, and nausea; a cardiovascular symptom such as tachycardia and blood pressure reduction; and a skin or mucous symptom such as itchiness or swelling of the eye, the lip, oral cavity, or the skin).

Examples of the allergic disease patient can include a pollinosis patient (patient sensitized to the pollen-derived allergen mentioned above), a food allergy patient (patient sensitized to the food-derived allergen mentioned above), a pollen-food allergy syndrome (PFAS) patient (patient sensitized to the pollen-derived allergen mentioned above and then sensitized to the food-derived allergen mentioned above), an insect allergy patient (patient sensitized to the insect-derived allergen mentioned above), a fungus allergy patient (patient sensitized to the fungus-derived allergen mentioned above), an animal allergy patient (patient sensitized to the animal-derived allergen mentioned above), a latex allergy patient (patient sensitized to the natural rubber-derived allergen mentioned above), and a latex-fruit syndrome patient (patient sensitized to the natural rubber-derived allergen mentioned above and then sensitized to the food-derived allergen mentioned above). A pollinosis patient or a PFAS patient is preferred.

The method for intranasally administering the present formulation for allergen immunotherapy can be any method of contacting the allergen-polysaccharide conjugate with nasal mucosa, and can be appropriately selected depending on the dosage form of the present formulation for allergen immunotherapy. Examples thereof can include: a method of dropwise addition to nasal mucosa; a method of spraying; a method of application using a cotton swab or the like; and a patch method. After administration of the present formulation for allergen immunotherapy, the nasal alae on both sides may be well massaged from above with fingers such that the allergen-polysaccharide conjugate is uniformly spread throughout the nasal mucosa.

The dose and the administration period of the allergen-polysaccharide conjugate contained in the present formulation for allergen immunotherapy are appropriately determined depending on age, body weight, sex, symptoms, sensitivity to the drug, and the like. The dose is, for example, in the range of doses of 1 µg to 200 mg/day (preferably doses of 0.1 to 100 mg/day, more preferably doses of 1 to 50 mg/day, further preferably doses of 1 to 30 mg/day, still further preferably doses of 5 to 20 mg/day), and the administration period is, for example, at least 10 days (preferably at least 12 days, more preferably at least 14 days). Since the present formulation for allergen immunotherapy enables the allergen-polysaccharide conjugate to be retained in nasal mucosa for a long time, a single dose per day is accepted. If necessary, the allergen-polysaccharide conjugate may be administered plural times (e.g., 2 to 4 times), and plural types of allergen-polysaccharide conjugates may be administered sequentially or concurrently. The allergen-polysaccharide conjugate contained in the present formulation for allergen immunotherapy may be administered daily (continuously administered), may be intermittently administered (e.g., intermittently administered such that a period from final administration to readministration is 2 to 180 days on average), or may be administered by a combination of daily administration and intermittent administration. AIT using the present formulation for allergen immunotherapy is unlikely to induce allergic symptoms as compared with conventional AIT. Therefore, the present formulation for allergen immunotherapy may be intermittently administered as maintenance therapy for several years to several tens of years.

Hereinafter, the present invention will be described more specifically with reference to Examples. However, the technical scope of the present invention is not limited by these examples.

### Example 1

### 1. Method

### [Preparation of white birch pollen-galactomannan conjugate]

White birch pollen with the largest number of PFAS patients was used as a pollen antigen. In order to prevent the IgE epitope of the white birch pollen from functioning, the white birch pollen was subjected to hypoallergenic treatment. Specifically, by the application of the method described in the document "Auris Nasus Larynx. 2014; 41 (4): 350-8", the white birch pollen was chemically conjugated with galactomannan, one of polysaccharides, through Maillard reaction to prepare a "white birch pollen-galactomannan conjugate". Since NOAFEL (no-observed-adverse-effect-level) of galactomannan is 5,200 mg/kg body weight/day (see the document "EFSA J. 2017; 15 (2): 4669"), 3.6% (7.2 mg in terms of a daily amount) of galactomannan was used in this experiment. A more specific method for preparing the "white birch pollen-galactomannan conjugate" will be shown below.

First, natural white birch pollen (harvested by the inventors from Nagano prefecture and Hokkaido, Japan) was mixed with a buffer solution and subjected to pulverization treatment using a pulverizer. Next, a supernatant was recovered after centrifugation, and the supernatant was passed through a 0.45 µm filter. The resultant was placed in a dialysis membrane, and after dialysis for 72 hours, the dialysate was transferred to a tray and powdered by freeze drying treatment. The dried powder was granulated with a mortar to prepare a white birch pollen crude extract antigen. The white birch pollen crude extract antigen and galactomannan (SUNFIBER HG, manufactured by Taiyo Kagaku Co., Ltd.) were mixed and dissolved at a weight ratio of 1:9 in water, then transferred to a tray, and powdered again by freeze drying, and the resulting powder was granulated to prepare a "white birch pollen crude extract antigen-galactomannan mixed powder". The "white birch pollen crude extract antigen-galactomannan mixed powder" was transferred to a thermo-hygrostat of 60°C and an inside humidity of 65% where Maillard reaction was performed for 14 days. Then, the powder was granulated with a mortar to prepare a "white birch pollen-galactomannan conjugate". The "white birch pollen-galactomannan conjugate" was confirmed by a pathogen test to be pathogen-free. Then, white petrolatum was added as an ointment base such that the final concentration of the "white birch pollen-galactomannan conjugate" was 4% to prepare a "white birch pollen-galactomannan conjugate"-containing ointment formulation. The "white birch pollen-galactomannan conjugate"-containing ointment formulation was enclosed in a container and preserved at ordinary temperature. Also, only galactomannan was transferred to a thermo-hygrostat of 60°C and an inside humidity of 65%, incubated for 14 days, and mixed with white petrolatum so as to attain a concentration of 4% contained. The resultant was used as a placebo.

### [Polyacrylamide electrophoresis]

3 types of samples ("white birch pollen-galactomannan conjugate", "white birch pollen crude extract antigen-galactomannan mixed powder", and galactomannan) were each dissolved into a concentration of 100 mg/mL in distilled water, and 3 µL each of the solutions was applied to each lane of "SuperSep(TM) ACE, 10-20% gel, 13 wells" (manufactured by FUJIFILM Wako Pure Chemical Corp.), followed by polyacrylamide electrophoresis (Figure 1).

### [ELISA method]

Blood was collected from 5 PFAS patients confirmed to contain *Betulaceae-specific* IgE in serum. Serum was prepared therefrom in accordance with a standard method and then added to a 96-well plate on which 4 types of antigens ("alder pollen crude extract antigen-galactomannan mixed powder" and "alder pollen-galactomannan conjugate", and "white birch pollen crude extract antigen-galactomannan mixed powder" and "white birch pollen-galactomannan conjugate") were immobilized. Then, 3 types of antibodies (HRP-labeled anti-human IgE antibody [diluted 1500-fold, manufactured by Invitrogen Corp.], HRP-labeled anti-human IgG antibody [diluted 3000-fold, manufactured by Bio-Rad Laboratories, Inc.], and HRP-labeled anti-human IgA antibody [diluted 3000-fold, manufactured by Bio-Rad Laboratories, Inc.]) were added thereto to perform an ELISA method (Figure 2). HRP activity was analyzed by using SpectraMax ABS/ABS Plus absorbance microplate reader (manufactured by Molecular Devices) to measure a value obtained by subtracting absorbance at a wavelength of 490 nm from absorbance (OD value) at 620 nm (OD value at 620 nm - OD value at 490 nm) (ordinate of Figure 2). The "alder pollen crude extract antigen-galactomannan mixed powder" was prepared by using natural alder pollen (produced in Yamagata prefecture, Japan; obtained from Shinjyo Mogami Organic Farmer Society Limited Company of Agricultural Production Corporation) instead of the natural white birch pollen in the method described in the item [Preparation of white birch pollen-galactomannan conjugate]. The "alder pollen-galactomannan conjugate" was prepared through the Maillard reaction of the "alder pollen crude extract antigen-galactomannan mixed powder" instead of the "white birch pollen crude extract antigen-galactomannan mixed powder" in the method described in the item [Preparation of white birch pollen-galactomannan conjugate].

### [BAT]

Using Allergenicity kit (manufactured by Beckman Coulter, Inc.), PFAS patient-derived blood collected with EDTA was mixed, together with 2 types of antigens (white birch pollen crude extract antigen and white birch pollen-galactomannan conjugate), into an activation buffer and incubated at 37°C for 10 minutes. Then, a reaction stopping solution and a hemolytic fixative were added thereto, and the mixture was incubated again at 37°C for 10 minutes. After centrifugation, a supernatant was applied to a flow cytometer (FACS Caliber, manufactured by Becton, Dickinson and Company) where CD294-positive cells (basophils) were developed to analyze the expression of CD203c (Figure 3).

### [Intranasal administration test in model animal]

100 µg of the white birch pollen crude extract antigen and an adjuvant (Alum, manufactured by Thermo Fisher Scientific Inc.) were subcutaneously administered once a week a total of three times to each BALB/c mouse (female, 8 to 12 weeks old) for sensitization. On week 3 (day 21), 3 types of antigens ("white birch pollen crude extract antigen-galactomannan mixed powder", "white birch pollen-galactomannan conjugate", and galactomannan) dissolved in saline were each intranasally administered at a dosage of 10 mg/30 µL for 5 days, and the mouse was euthanized by cervical dislocation 4 hours after final administration. The number of nasal rubs by the mouse was also measured for 10 minutes immediately after final administration (Figure 4A). Since the mouse had a small nasal cavity, the 3 types of antigens dissolved in saline instead of an ointment were used. A nasal tissue specimen was prepared in accordance with a standard method, and a double immunohistological staining method using an anti-Bet v1 antibody (manufactured by Novus Biologicals) and an anti-CD11c antibody (manufactured by RayBiotech, Inc.) was then performed in accordance with a standard method.

### [Intranasal administration test in human]

A total of 10 healthy males and females aged 18 and over and 64 and under having no *Betulaceae* pollen sensitization were selected as test subjects from January to March, 2022 and assigned to a "white birch pollen-galactomannan conjugate"-containing ointment formulation administration group involving 8 subjects and a placebo administration group involving 2 subjects by a random double-blind method (Table 1). Anterior rhinoscopic findings of the test subjects were observed by an otolaryngologist before intranasal administration to confirm the absence of macroscopic abnormality.

Initial intranasal administration was performed as follows: approximately 0.1 g each of the "white birch pollen-galactomannan conjugate"-containing ointment formulation and the placebo ointment was added to the tip of a cotton swab (Figure 5A(1)), and the ointments were applied to the nasal mucosa of the nasal cavity on one side of the "white birch pollen-galactomannan conjugate"-containing ointment formulation administration group and the placebo administration group, respectively (Figures 5A(2) and 5B), while an ointment containing 12.5 mg of an artificial sweetener (saccharin) was applied to the nasal mucosa of the nasal cavity on the other side of both the groups using a cotton swab with the ointment (Figures 5A(3) and 5B). Then, the nasal alae on both sides were well massaged from above with fingers such that the ointments were uniformly spread throughout the nasal mucosa (Figure 5A(4)). No appearance of immediate-type symptoms was confirmed for 5 minutes. A time to perceive sweet taste after intranasal administration and a time to finish perceiving the sweet taste (duration) were each measured and also confirmed to have little difference between both the groups. For the white birch pollen-galactomannan conjugate-containing ointment formulation administration group, the time to perceive sweet taste was 33.9 minutes on average (ranging from 5 to 77 minutes), and the time to finish perceiving the sweet taste was 77.6 minutes on average (ranging from 15 to 315 minutes). Consideration of the fact that the sublingual mucous retention time of an allergen in sublingual immunotherapy is usually 1 to 2 minutes indicates that intranasal immunotherapy has a longer retention time of an allergen in mucosa than that of sublingual immunotherapy.

The second and later doses of intranasal administration for a total of 14 days were performed as follows: approximately 0.1 g each of the "white birch pollen-galactomannan conjugate"-containing ointment formulation and the placebo ointment was added to the tip of a cotton swab (Figure 5A(1)), and the ointments were applied to the nasal mucosa of the nasal cavity on one side of the "white birch pollen-galactomannan conjugate"-containing ointment formulation administration group and the placebo administration group, respectively (Figures 5A(2) and 5B). Then, approximately 0.1 g each of the "white birch pollen-galactomannan conjugate"-containing ointment formulation and the placebo ointment was also applied to the nasal mucosa of the nasal cavity on the other side of both the groups, respectively (Figures 5A(3) and 5B). Then, the nasal alae on both sides were well massaged from above with fingers such that the ointments were uniformly spread throughout the nasal mucosa (Figure 5A(4)).

A serum total IgE concentration (Table 2), the number of peripheral blood eosinophils (Table 2), the proportion of a test subject who exhibited a serum alder-specific IgE concentration of a detection limit (less than 0.10) (Table 3), the proportion of a test subject who exhibited a serum white birch-specific IgE concentration of a detection limit (less than 0.10) (Table 3), the proportion of a test subject who exhibited a serum Bet v1-specific IgE concentration of a detection limit (less than 0.1) (Table 3), a serum white birch-specific IgG concentration (Figure 6), a saliva white birch-specific IgA concentration (Figure 7A), and a saliva white birch-specific IgG concentration (Figure 7B) were measured for the test subjects before intranasal administration, 14 days after the start of intranasal administration, and 84 days after the start of intranasal administration. Specifically, the serum total IgE concentration, the proportion of a test subject who exhibited a serum alder-specific IgE concentration of a detection limit (less than 0.10), the proportion of a test subject who exhibited a serum white birch-specific IgE concentration of a detection limit (less than 0.10), the proportion of a test subject who exhibited a serum Bet v1-specific IgE concentration of a detection limit (less than 0.1) were each measured using ImmunoCAP (manufactured by Thermo Fisher Scientific Inc.). The number of peripheral blood eosinophils was measured by a flow cytometry method in accordance with a standard method. The serum white birch-specific IgG concentration, the saliva white birch-specific IgA concentration, and the saliva white birch-specific IgG concentration were analyzed by using SpectraMax ABS/ABS Plus absorbance microplate reader to measure "OD value at 620 nm - OD value at 490 nm" (ordinates of Figures 6 and 7) in accordance with the method described in the item [ELISA method]. The saliva white birch-specific IgA concentration and the saliva white birch-specific IgG concentration were also analyzed 7 days after the start of intranasal administration. Anterior rhinoscopic findings were observed again by an otolaryngologist 14 days after the start of intranasal administration to confirm the absence of macroscopic abnormality in the nasal mucosa of the test subjects.

**[Table 1]**

| Item | "White birch pollen-galactomannan conjugate"-containing ointment formulation administration group | Placebo administration group |
|---|---|---|
| Male/female (the number of people) | 4/4 | 2/0 |
| Average age (years old) (±SD) | 31.3 (8.6) | 35.0 (4.2) |
| Average height (cm) (±SD) | 166.65 (8.66) | 174.74 (0.28) |
| Average body weight (kg) (±SD) | 66.16 (15.03) | 86.75 (13.93) |
| Having no complication | 8 (100%) | 2 (100%) |
| Having no past history | 6 (75%) | 1 (50%) |
| Having past history | 2 (25%) | 1 (50%) |
| Having no history of present illness | 8 (100%) | 2 (100%) |
| Having no concurrent medication | 8 (100%) | 2 (100%) |
| Average nonspecific IgE before intranasal administration (IU/mL) | 50.88 | 27.50 |
| Average number of peripheral blood eosinophils before intranasal administration (the number of eosinophils/µL) | 90.38 | 12.00 |
| Negative for the number of nasal discharge eosinophils before intranasal administration | 8 (100%) | 2 (100%) |
| Having no wheal in skin prick test | 8 (100%) | 2 (100%) |

### 2. Results

### [Confirmation of conjugation in white birch pollen-galactomannan conjugate]

In order to confirm conjugation in the "white birch pollen-galactomannan conjugate", 3 types of samples ("white birch pollen-galactomannan conjugate", "white birch pollen crude extract antigen-galactomannan mixed powder", and galactomannan) were subjected to polyacrylamide electrophoresis.

As a result, a Bet v1-derived band detected in the "white birch pollen crude extract antigen-galactomannan mixed powder" disappeared in the "white birch pollen-galactomannan conjugate" (Figure 1).

This result indicates that in the "white birch pollen-galactomannan conjugate" sample, most of the white birch pollen forms a conjugate with galactomannan.

### [Confirmation of hypoallergenicity in Betulaceae pollen-galactomannan conjugate]

Next, in order to confirm hypoallergenicity in the *Betulaceae* pollen (such as white birch pollen)-galactomannan conjugates, PFAS patient-derived serum containing Betulaceae-specific IgE was added as a primary antibody to wells on which 4 types of antigens ("alder pollen crude extract antigen-galactomannan mixed powder" and "alder pollen-galactomannan conjugate", and "white birch pollen crude extract antigen-galactomannan mixed powder" and "white birch pollen-galactomannan conjugate") were immobilized. Then, 3 types of antibodies (HRP-labeled anti-human IgE antibody, HRP-labeled anti-human IgG antibody, and HRP-labeled anti-human IgA antibody) were added thereto to perform an ELISA method.

As a result, in the case of using the HRP-labeled anti-human IgG antibody as a secondary antibody, HRP activity was rarely different between the "alder pollen crude extract antigen-galactomannan mixed powder" and the "alder pollen-galactomannan conjugate" (Figure 2B). In the case of using the HRP-labeled anti-human IgA antibody as a secondary antibody, HRP activity was also rarely different between the "white birch pollen crude extract antigen-galactomannan mixed powder" and the "white birch pollen-galactomannan conjugate" (Figure 2C). By contrast, in the case of using the HRP-labeled anti-human IgE antibody as a secondary antibody, HRP activity was drastically reduced in the "alder pollen-galactomannan conjugate" compared with the "alder pollen crude extract antigen-galactomannan mixed powder" (Figure 2A).

These results indicate that the *"Betulaceae* pollen (such as alder pollen and white birch pollen)-galactomannan conjugates" exhibit reduced binding activity against *Betulaceae-specific* IgE (i.e., are hypoallergenic) while maintaining binding activity against *Betulaceae-specific* IgG or IgA, which is an antagonistic antibody for IgE.

In order to further confirm hypoallergenicity in the *"Betulaceae* pollen-galactomannan conjugates", PFAS patient-derived basophils were incubated in the presence of 2 types of antigens ("white birch pollen crude extract antigen-galactomannan mixed powder" and "white birch pollen-galactomannan conjugate") to perform BAT.

As a result, when the PFAS patient-derived basophils were incubated in the presence of the "white birch pollen crude extract antigen-galactomannan mixed powder", the proportion of basophils positive for a basophil activation cell surface marker CD203c (CD294-positive cells) was as high as the same level as that of a positive control (anti-IgE antibody) (Figures 3C and 3D). By contrast, when the PFAS patient-derived basophils were incubated in the presence of the "white birch pollen-galactomannan conjugate", it was found that the proportion was decreased to the same level as that of a negative control (PBS) (Figures 3A and 3B).

These results indicate that the "white birch pollen-galactomannan conjugate" does not activate basophils and is hypoallergenic because it can be predicted that the "white birch pollen-galactomannan conjugate" does not bind to a specific IgE antibody on basophils.

### [Confirmation of usefulness of white birch pollen-galactomannan conjugate as formulation for allergen immunotherapy to be used in intranasal administration - (1)]

Next, in order to confirm usefulness of the "white birch pollen-galactomannan conjugate" as a formulation for allergen immunotherapy to be used in intranasal administration, 3 types of antigens ("white birch pollen crude extract antigen-galactomannan mixed powder", "white birch pollen-galactomannan conjugate", and galactomannan) were intranasally administered to mice sensitized to the white birch pollen crude extract antigen, and the number of nasal rubs was measured. In addition, nasal tissue specimens of the mice were subjected to a double immunohistological staining method using an anti-Bet v1 antibody and an anti-CD11c antibody.

As a result, it was found that when the "white birch pollen-galactomannan conjugate" was intranasally administered to the mouse sensitized to the white birch pollen crude extract antigen, the number of nasal rubs was significantly decreased as compared with the case where the "white birch pollen crude extract antigen-galactomannan mixed powder" was intranasally administered thereto (Figure 4A). It was also found that large cells in the lamina propria near nasal-associated lymphoid tissues (NALTs) of the mouse given the intranasally administered "white birch pollen-galactomannan conjugate" were anti-CD11c antibody-positive dendritic cells and were also positive for the anti-Bet v1 antibody (Figure 4B); and the anti-CD11c antibody-positive dendritic cells in some NALTs were positive for the anti-Bet v1 antibody (arrow of Figure 4B).

These results indicate that when the "white birch pollen-galactomannan conjugate" is intranasally administered, dendritic cells are able to present the white birch pollen in the white birch pollen-galactomannan conjugate as an antigen, as in a usual white birch pollen antigen, while allergic symptoms are minimized, and indicate that the "white birch pollen-galactomannan conjugate" is useful as a formulation for allergen immunotherapy to be used in intranasal administration.

### [Confirmation of usefulness of white birch pollen-galactomannan conjugate as formulation for allergen immunotherapy to be used in intranasal administration - (2)]

In order to further confirm that the "white birch pollen-galactomannan conjugate"-containing ointment formulation can be administered safely and conveniently and is useful as a formulation for allergen immunotherapy to be used in intranasal administration, the "white birch pollen-galactomannan conjugate"-containing ointment formulation or the placebo was intranasally administered to test subjects having no *Betulaceae* pollen sensitization, and each test on allergic symptoms was performed.

As a result, both the test subjects given the intranasally administered "white birch pollen-galactomannan conjugate"-containing ointment formulation (i.e., the "white birch pollen-galactomannan conjugate"-containing ointment formulation administration group) and the test subjects given the intranasally administered placebo (i.e., the placebo administration group) exhibited no suspected symptom of allergic rhinitis, such as nasal discharge, sneeze, nasal congestion, or pruritus, during the administration period and also exhibited no suspected finding of the appearance of allergic rhinitis from macroscopic observation of nasal mucosa. In both the groups, the serum total IgE concentration and the number of peripheral blood eosinophils were rarely different between before and after administration (Table 2), and no test subject was found positive for serum white birch-specific IgE, serum alder-specific IgE, and serum Bet v1-specific IgE (Table 3).

On the other hand, the serum white birch-specific IgG concentration was rarely different between before and after administration in the placebo administration group, whereas significant elevation was observed 84 days after the start of intranasal administration in the "white birch pollen-galactomannan conjugate"-containing ointment formulation administration group (Figure 6). Likewise, the saliva white birch-specific IgA concentration and the saliva white birch-specific IgG concentration were also rarely different between before and after administration in the placebo administration group, whereas significant elevation was observed 14 days after the start of intranasal administration in the "white birch pollen-galactomannan conjugate"-containing ointment formulation administration group (Figure 7).

These results indicate that the "white birch pollen-galactomannan conjugate", when intranasally administered, can increase white birch-specific IgG and IgA in saliva or blood while minimizing allergic symptoms. It is thus expected that this conjugate can treat pollinosis and PFAS mainly having OAS symptoms. Furthermore, the administration method is very convenient and involves no pain. All the test subjects were able to learn the manipulation by one guidance, and no test subject produced discomfort as to usability.

**[Table 2]**

| Testing item | "White birch pollen-galactomannan conjugate"-containing ointment formulation administration group | Placebo administration group |
|---|---|---|
| Serum total IgE (IU/mL) | | |
| Before intranasal administration | 50.9 (54.2) | 27.5 (27.6) |
| 14 days after start of intranasal administration | 53.3 (57.6) | 23.5 (23.3) |
| 84 days after start of intranasal administration | 63.9 (82.4) | 24.0 (24.0) |

| The number of peripheral blood eosinophils (/µL) | | |
|---|---|---|
| Before intranasal administration | 90.4 (96.9) | 128.0 (118.8) |
| 14 days after start of intranasal administration | 110.0 (110.7) | 76.5 (108.2) |
| 84 days after start of intranasal administration | 77.1 (115.7) | 91.5 (51.6) |

The values within the parentheses in the table denote standard deviation (±SD).

**[Table 3]**

| Testing item | "White birch pollen-galactomannan conjugate"-containing ointment formulation administration group | Placebo administration group |
|---|---|---|
| Serum alder-specific IgE of detection limit (less than 0.10) (UA/mL) | | |
| Before intranasal administration | 8 (100%) | 2 (100%) |
| 14 days after start of intranasal administration | 8 (100%) | 2 (100%) |
| 84 days after start of intranasal administration | 8 (100%) | 2 (100%) |

| Serum white birch-specific IgE of detection limit (less than 0.10) (UA/mL) | | |
|---|---|---|
| Before intranasal administration | 8 (100%) | 2 (100%) |
| 14 days after start of intranasal administration | 8 (100%) | 2 (100%) |
| 84 days after start of intranasal administration | 8 (100%) | 2 (100%) |

| Serum Bet v1-specific IgE of detection limit (less than 0.1) (UA/mL) | | |
|---|---|---|
| Before intranasal administration | 8 (100%) | 2 (100%) |
| 14 days after start of intranasal administration | 8 (100%) | 2 (100%) |
| 84 days after start of intranasal administration | 8 (100%) | 2 (100%) |

### Example 2

In Example 1 described above, the "white birch pollen-galactomannan conjugate"-containing ointment formulation was confirmed to induce white birch-specific IgA and IgG without inducing the onset of allergic rhinitis and white birch-specific IgE, when intranasally administered to test subjects having no *Betulaceae* pollen sensitization. Next, whether to exert alleviating action on allergic symptoms was examined when the "white birch pollen-galactomannan conjugate" was intranasally administered to PFAS patients, specifically, patients with apple allergy caused by *Betulaceae* pollen sensitization (i.e., patients who were positive for serum alder-specific IgE or serum white birch-specific IgE and exhibited an oral allergic symptom ascribable to raw apple).

### 1. Test subject

The patients with apple allergy caused by *Betulaceae* pollen sensitization (PFAS patient), specifically, patients having the patient background shown in Tables 4 and 5 (n = 5) were used as test subjects. The test subjects excluded, for example, 1) patients having a history of anaphylaxis to raw apple, 2) patients who were treated by allergen immunotherapy with *Betulaceae* pollen in the past, 3) patients who were using an immunosuppressant by receiving a biological formulation or steroid administration other than external use or inhalation, 4) pregnant women or women of childbearing potential, and 5) breast-feeding women.

**[Table 4]**

| Item | Before intranasal administration |
|---|---|
| Male/Female (the number of people) | 1/4 |
| Average age (years old) (±SD) (range) | 47.8(±12.8) (33 to 63) |
| Average age of onset (years old) (range) | 18.8 (12 to 27) |
| Average disease duration (years) (range) | 28.8 (13 to 51) |
| Peripheral blood eosinophil (the number of eosinophils/µL) (±SD) (range) | 212.6.(±113.4) (114 to 403) |
| Serum total IgE (IU/mL) (±SD) (range) | 867.2(±934.0) (91 to 2118) |
| Serum white birch -specific IgE (UA/mL) (±SD) (range) | 39.724(±28.304) (3.12 to 71.10) |
| Serum Bet v1-specific IgE (UA/mL) (±SD) (range) | 51.578(±38.695) (3.59 to 88.30) |
| Serum alder-specific IgE (UA/mL) (±SD) (range) | 37.082(±29.709) (3.31 to 72.20) |

**[Table 5]**

| Skin prick test | Before intranasal administration |
|---|---|
| Average wheal diameter (mm) | |
| Positive control (±SD) | 4.14(0.80) |
| Negative control (±SD) | 0.00(±0.00) |
| "White birch pollen-galactomannan conjugate" (±SD) | 0.00(±0.00) |
| Raw apple (±SD) | 4.52(±1.52) |
| Untreated white birch pollen (±SD) | 8.82(±4.41) |
| Raw apple/positive control ratio (±SD) | 1.1621 (±0.4798) |
| Untreated white birch pollen/positive control ratio (±SD) | 2.2383(±1.2897) |

### 2. Method

### [Intranasal administration test in human]

The "white birch pollen-galactomannan conjugate"-containing ointment formulation prepared in Example 1 described above was intranasally administered to the test subjects in accordance with the method described in the item [Intranasal administration test in human] of Example 1 described above. The intranasal administration was performed daily up to 14 days after the start of intranasal administration and performed once a week from 15 days to 84 days after the start of intranasal administration.

### [Oral challenge test with raw apple]

An oral challenge test using varying amounts (2 g, 4 g, 8 g, 16 g, 32 g, or 64 g) of raw apple (oral challenge test with raw apple) was carried out for the test subjects before intranasal administration and 84 days after the start of intranasal administration in accordance with the method described in Japanese Guidelines for Food Allergy 2021 (Food Allergy Committee of Japanese Society of Pediatric Allergy and Clinical Immunology), and an ingestion threshold, a total intake, and VAS were measured.

### [Wheal diameter in skin prick test with raw apple and white birch extract]

One drop each of 5 types of test substances ("white birch pollen-galactomannan conjugate"-containing ointment formulation ("white birch pollen-galactomannan conjugate"); Allergen Scratch Extract Control Liquid "Torii" or saline (negative control); Allergen Scratch Extract Positive Control Liquid "Torii" Histamine Dihydrochloride (positive control); grated raw apple (raw apple); and white birch extract (manufactured by ALK-Abello A/S, 1:200) (untreated white birch pollen)) was applied to the forearm skin of the test subjects before intranasal administration and then injected in a very small amount to the skin using a bifurcated needle or a prick lancet needle, and the presence or absence of a wheal and an average wheal diameter were measured 15 minutes later (Table 5). Also, one drop each of 3 types of test substances (positive control, raw apple, and untreated white birch pollen) was applied to the forearm skin of the test subjects before intranasal administration and 84 days after the start of intranasal administration and then injected in a very small amount to the skin using a bifurcated needle or a prick lancet needle, and an average wheal diameter of the raw apple based on the positive control (raw apple/positive control ratio) and an average wheal diameter of the untreated white birch pollen based on the positive control (untreated white birch pollen/positive control ratio) were measured 15 minutes later (Table 6).

### [Measurement of various substances in serum]

Various IgE concentrations in serum (serum white birch-specific IgE concentration [Table 4 and Figure 9A], serum alder-specific IgE concentration [Table 4 and Figure 9B], and serum Bet v1-specific IgE concentration [Table 4 and Figure 9C], and serum total IgE concentration [Table 4 and Figure 9D]) were measured for the test subjects before intranasal administration, 14 days after the start of intranasal administration, and 84 days after the start of intranasal administration using ImmunoCAP (manufactured by Thermo Fisher Scientific Inc.). The number of peripheral blood eosinophils (Table 4 and Figure 9E) was measured by a flow cytometry method in accordance with a standard method. A serum white birch-specific IgG concentration (ordinate of Figure 10) was analyzed by using SpectraMax ABS/ABS Plus absorbance microplate reader to measure "OD value at 620 nm - OD value at 490 nm" in accordance with the method described in the item [ELISA method] of Example 1 described above.

### 3. Results

### [Oral challenge test with raw apple]

When the "white birch pollen-galactomannan conjugate"-containing ointment formulation was intranasally administered to patients with apple allergy caused by *Betulaceae* pollen sensitization, it was found that an average value of ingestion thresholds (i.e., weight thresholds of apple that induced oral allergic symptoms) in the patients 84 days after the start of intranasal administration was 64 [±0.00]) g, which was increased drastically and significantly as compared with an average value of ingestion thresholds (2.8 [±1.10] g) in the patients before intranasal administration (Figure 8A). It was also found that an average value of total intakes (i.e., total intakes of apple that was able to be orally ingested) in the patients 84 days after the start of intranasal administration was 126 [±0.00]) g, which was increased drastically and significantly as compared with an average value of total intakes (3.6[±2.19] g) in the patients before intranasal administration (Figure 8B). It was further found that an average value of VAS in the patients 84 days after the start of intranasal administration was 0.000 (±0.000) mm, which was decreased drastically and significantly as compared with an average value of VAS (26.70 [±14.72] mm) in the patients before intranasal administration (Figure 8C) .

These results indicate that the "white birch pollen-galactomannan conjugate", when intranasally administered to patients with apple allergy caused by *Betulaceae* pollen sensitization (PFAS patients), drastically improved allergic symptoms to apple in the patients and as a result, drastically increased the amount of orally ingestible apple.

### [Wheal diameter in skin prick test with raw apple and white birch extract]

When the "white birch pollen-galactomannan conjugate"-containing ointment formulation was intranasally administered to patients with apple allergy caused by *Betulaceae* pollen sensitization (PFAS patients), it was found that a raw apple/positive control ratio (i.e., an average wheal diameter of the raw apple based on the positive control) in the patients 84 days after the start of intranasal administration was 0.9862 (±0.33969), which was decreased as compared with a raw apple/positive control ratio (1.1621 [±0.4798]) in the patients before intranasal administration (Table 6). It was also found that an untreated white birch pollen/positive control ratio (i.e., an average wheal diameter of the untreated white birch pollen based on the positive control) in the patients 84 days after the start of intranasal administration was 1.6090 (±0.6247), which was decreased as compared with an untreated white birch pollen/positive control ratio (2.2383 [±1.2897]) in the patients before intranasal administration (Table 6).

These results indicate that the "white birch pollen-galactomannan conjugate", when intranasally administered to patients with apple allergy caused by *Betulaceae* pollen sensitization (PFAS patients), improves allergic symptoms not only to apple but to white birch pollen in the patients.

Since *Fagaceae* pollen and *Betulaceae* pollen are known to have cross antigenicity, use of the present formulation for allergen immunotherapy is well expected to also improve allergic symptoms in *Fagaceae* pollinosis patients or *Fagaceae* pollen-food allergy syndrome patients.

**[Table 6]**

| Skin prick test | Before intranasal administration | 84 days after start of intranasal administration |
|---|---|---|
| Average wheal diameter (mm) | | |
| Raw apple/positive control ratio (±SD) | 1.1621(±0.4798) | 0.9862(±0.33969) |
| Untreated white birch pollen/positive control ratio (±SD) | 2.2383(±1.2897) | 1.6090(±0.6247) |

### [Measurement of various substances in serum]

When the "white birch pollen-galactomannan conjugate"-containing ointment formulation was intranasally administered to patients with apple allergy caused by *Betulaceae* pollen sensitization (PFAS patients), a serum white birch-specific IgE concentration (Figure 9A), a serum Bet v1-specific IgE concentration (Figure 9C), a serum total IgE concentration (Figure 9D), and the number of peripheral blood eosinophils (Figure 9E) were rarely changed from those before intranasal administration, and a serum alder-specific IgE concentration (Figure 9B) was rather significantly decreased 84 days after the start of intranasal administration. The patients exhibited few suspected symptoms of allergic rhinitis, such as nasal discharge, sneeze, nasal congestion, or pruritus, during the administration period. On the other hand, a serum white birch-specific IgG concentration was significantly elevated 14 days after the start of intranasal administration, and the elevated value was maintained up to at least 84 days after the start of administration (Figure 10).

These results indicate that the "white birch pollen-galactomannan conjugate", when intranasally administered to patients with apple allergy caused by *Betulaceae* pollen sensitization (PFAS patients), can increase white birch-specific IgG in blood while minimizing allergic symptoms.

For 3 months after the completion of the intranasal administration test, it was also confirmed that all the 5 test subjects were able to eat raw apple or raw pear without allergic symptoms even when the intranasal administration test (i.e., allergen immunotherapy) was not performed.

This result indicates that use of the present formulation for allergen immunotherapy enables a patient not only to acquire a desensitized state of allergic response (i.e., a state in which no allergic symptom caused by the allergen appears as long as the ingestion of the allergen-polysaccharide conjugate contained in the present formulation for allergen immunotherapy is continued), but to acquire a sustained unresponsive state of allergic response (i.e., a state in which even if the allergen-polysaccharide conjugate contained in the present formulation for allergen immunotherapy is not ingested for several weeks to several months, no allergic symptom is induced by subsequent ingestion of the allergen), and enables true immune tolerance (tolerance without allergic symptoms) to be acquired.

Although the test subjects also included a test subject with intercurrent tofu (soybean) allergy and bean sprout allergy, a test subject with cherry allergy, and a test subject with peach allergy, these test subjects were also confirmed to be able to ingest any of the foods without allergic symptoms after the completion of the intranasal administration test.

This result indicates that any food, allergy of which is derived from *Betulaceae* sensitization, can resolve without being limited to apple.

### Industrial Applicability

The present invention contributes to allergen immunotherapy.

## Claims

1. A formulation for allergen immunotherapy, comprising an allergen-polysaccharide conjugate, wherein the formulation for allergen immunotherapy is intranasally administered to an allergic disease patient.

2. The formulation for allergen immunotherapy according to claim 1, wherein the formulation for allergen immunotherapy is an ointment formulation.

3. The formulation for allergen immunotherapy according to claim 1, wherein the polysaccharide is galactomannan.

4. The formulation for allergen immunotherapy according to any one of claims 1 to 3, wherein the allergen is a pollen-derived allergen.

5. The formulation for allergen immunotherapy according to claim 4, wherein the allergic disease patient is a pollinosis patient or a pollen-food allergy syndrome patient.

6. The formulation for allergen immunotherapy according to claim 5, wherein the pollen is *Fagales* pollen.

7. The formulation for allergen immunotherapy according to claim 6, wherein the *Fagales* pollen is *Betulaceae* pollen or *Fagaceae* pollen.

8. The formulation for allergen immunotherapy according to claim 7, wherein the *Betulaceae* pollen is white birch pollen or alder pollen.
